# EUROPÄISCHE PATENTSCHRIFT

(11) **EP 0 937 033 B1**
(45) Veröffentlichungstag und Bekanntmachung des Hinweises auf die Patenterteilung: **28.08.2002**
(21) Anmeldenummer: 97913174.5
(22) Anmeldetag: 24.10.1997
(51) Int. Cl.: C07C 263/20

(54) **VERFAHREN ZUR ISOMERENTRENNUNG VON ISOCYANATEN**
PROCESS FOR THE ISOMERIC SEPARATION OF ISOCYANATES
PROCEDE DE SEPARATION ISOMERIQUE D'ISOCYANATES

(30) Priorität: 06.11.1996 DE 19645659
(43) Veröffentlichungstag der Anmeldung: 25.08.1999
(73) Patentinhaber: BAYER AG, 51368 Leverkusen (DE)
(72) Erfinder: DROPE, Rüdiger, D-50767 Köln (DE); GRENNER, Dieter, D-51373 Leverkusen (DE); HETZEL, Hartmut, D-50858 Köln (DE); SCHAL, Hans-Peter, D-41539 Dormagen (DE); WEGENER, Gerhard, D-40822 Mettmann (DE)
(86) Internationale Anmeldenummer: EP9705884
(87) Internationale Veröffentlichungsnummer: WO98019993

(56) Entgegenhaltungen:
- EP-A- 0 023 015
- EP-A- 0 097 243
- US-A- 3 872 009
- US-A- 4 787 985
- CHEMICAL ABSTRACTS, vol. 100, no. 24, 11.Juni 1984 Columbus, Ohio, US; abstract no. 192323k, N. A. POGODIN ET AL.: "Separation of diphenylmethane diisocyanate isomers by crystallization" Seite 2; Spalte 1; XP002052778 & DEPOSITED DOC. (SPSTL 298 KHP-D82), 1982, MOSKOW, USSR,
- CHEMICAL ABSTRACTS, vol. 102, no. 6, 11.Februar 1985 Columbus, Ohio, US; abstract no. 47895k, O. FISCHER ET AL.: "Purification of compounds forming eutectics and solid solutions by fractional crystallization " Seite 119; Spalte 2; XP002052779 & PROCESS TECHNOL. PROC., 1984, Seiten 153-157,
- Sattler, Thermische Trennverfahren, VCH-Verlag 1988, Seiten 529-531, Tabelle 7-8

## Beschreibung

Die vorliegende Erfindung betrifft ein Verfahren zur Trennung von Isocyanatisomeren durch Suspensionskristallisation.

Toluylendiisocyanat (TDI) fällt während der Herstellung im allgemeinen als eine Mischung aus 80 Gew.-% Toluylen-(2,4)-diisocyanat (2,4-TDI) und 20 Gew.-% Toluylen-(2,6)-diisocyanat (2,6-TDI) an.

Aufgrund der unterschiedlichen Eigenschaften, insbesondere der Reaktionsgeschwindigkeit gegenüber Polyolen bei der Polyadditionsreaktion zur Herstellung von Polyurethanen wird neben dem Einsatz des aus dem Herstellungsprozeß resultierenden Isomerengemisches (T80) sowohl reines 2,4-TDI (T100) als auch ein an 2,4-TDI abgemageites Isomerengemisch mit einem 2,4-TDI-Gehalt von 65 bis 68 Gew.-% (T65) technisch eingesetzt.

Aufgrund der unterschiedlichen Geometrie der Molekülstrukturen der beiden Isomeren zeigt das Phasendiagramm auf der 2,4-TDI-Seite eine deutliche Gefrierpunktserniedrigung. Der Gefrierpunkt des Isomerengemisches 80:20 beträgt 13,6°C und der des Isomerengemisches 65:35 nur noch 5°C. In dem gesamten Mischungsbereich zwischen 100 und 65 Gew.-% 2,4-TDI kristallisiert beim iangsamen Abkühlen unter die Liquidus-Temperatur jeweils über 99 %iges T100 aus.

Ähnliche Verhältnisse liegen beim Diphenylmethan-Diisocyanat (MDI) vor. Es fällt als Mischung von 4,4'-MDI und 2,4'-MDI im Verhältnis zwischen 70:30 bis 90:10 an. Der Schmelzpunkt von 4,4-MDI liegt bei 40°C, der einer Mischung von 60 % 4,4-MDI und 40 % 2,4-MDI liegt bei 21 bis 23°C je nach zusätzlichem Gehalt der Mischung an 2,2-MDI im Bereich bis 2 %.

Die Kristallisation aus den entsprechenden Mischungen ist daher ein geeignetes Verfahren für die Isomerentrennung.

EP-A-23 015 offenbart ein Verfahren zur Abtrennung von 2,4-Toluylendiisocyanat (2,4-TDI) aus einem im Wesentlichen 2,4- und 2,6-TDI enthaltenden Isomerengemisch. Die Gewinnung des Kristallisates erfolgt durch tentrifugieren nach einer Rekristallisation im "slurry tank" über mehrere stunden zur Um kristallisation Kleinster Kristallpartikel.

Als übliches Isomerentrennungsverfahren hat sich das statische Kristallisationsverfahren durchgesetzt, bei dem das zu isolierende Isomere an feststehenden gekühlten Flächen abgeschieden, anschließend von der "Mutterlauge" getrennt und wieder aufgeschmolzen wird.

Energetisch günstiger und mit besserem Trenneffekt als statische Kristallisationsverfahren lassen sich im allgemeinen Kristallisationsverfahren in Suspension durchführen, wenn für die Abtrennung und das Aufschmelzen der Kristallphase sogenannte Waschsäulen gemäß US-A 3 777 892 und US-A 3 872 009 eingesetzt werden.

Grundsätzlich sind solche Suspensionskristallisationsverfahren insbesondere für wäßrige Systeme, wie z.B. den Entzug von Wasser aus Lebensmittel Flüssigkeiten, aus den US-A 4 004 886, US-A 4 316 368, US-A 4 430 104 und US-A 4 459 144 bekannt. Ferner wurden gemäß US-A 4 787 985 Suspensionskristallisationsverfahren für die Reinigung von Chemikalien vorgeschlagen.

Allen diesen Verfahren gemeinsam ist, daß sie (bei mehrstufiger Kristallisation je Stufe) einen Kristallisationsbehälter, in dem durch Wärmeentzug Kristalle ausgefroren werden, einen Rekristallisationsbehälter, in dem größere Kristalle zu Lasten der kleineren wachsen, und einen Filter, in dem die Kristalle von der Mutterlauge getrennt werden, aufweisen. Zumindest der Filter der letzten Stufe ist als Waschsäule ausgebildet.

Aufgrund der nur verschwindend geringen Festphasenlöslichkeit von 2,6-TDI in 2,4-TDI bzw. 2,4-MDI in 4,4-MDI sind einstufige Suspensionskristallisationsverfahren für die Isomerentrennung an sich geeignet.

Probleme bereitet jedoch die Rekristallisation im Rekristallisationsbehälter, d.h. die Kristallvergrößerung, aufgrund der geringen Diffusionsgeschwindigkeit der Moleküle in der flüssigen Phase. Da der Stoffaustausch an der Kristalloberfläche durch die Diffusionsgeschwindigkeit in der Flüssigkeitsgrenzschicht bestimmt ist, würden unwirtschaftlich lange Rekristallisationszeiten erforderlich mit entsprechend großen Rekristallisationsbehältern.

Probleme bereiten ferner die länglichen, fast nadelförmigen 2,4-TDI-Kristalle, die einerseits eine stark herabgesetzte Rekristallisationsgeschwindigkeit bedingen und andererseits in der Waschsäule nur schwer abtrennbar sind.

Es wurde nun gefunden, daß das Suspensionskristallisationsverfahren für die Isomerentrennung von Isocyanaten, insbesondere TDI und MDI, dann vorteilhaft eingesetzt werden kann, wenn vor der Einleitung des Kristallbreis in die Waschkolonne 5 bis 40, vorzugsweise 10 bis 40, insbesondere bevorzugt 15 bis 30 Gew.-% der zu isolierenden Isomeren-Kristalle unter gleichzeitiger Erhöhung der Temperatur des Kristallbreis um 4,5 bis 9°C wieder aufgeschmolzen werden.

Gegenstand der vorliegenden Erfindung ist demgemäß ein Verfahren zur Gewinnung von reinen Isocyanat-Isomeren, insbesondere 2,4-TDI bzw. 4,4-MDI, aus einer entsprechenden Isomeren-Mischung durch Suspensionskristallisation und Abtrennung der reinen Kristalle, das dadurch gekennzeichnet ist, daß vor der Abtrennung der Kristalle 5 bis 40 Gew.-% der erzeugten Kristalle unter gleichzeitiger Erhöhung der Temperatur des Kristallbreies um 4,5 bis 9°C wieder aufgeschmolzen werden und die Abtrennung der isomeren reinen Kristalle aus der suspension unter Aufschmelzen in einer Waschsäule erfolgt.

Erfindungs gemäß erfolgt das Wiederaufschmelzen der Kristalle in einem Mischbehälter, der der Waschsäule vorgeschaltet ist und in dem eine Temperatur aufrecht erhalten wird, die 4,5 bis 9°C über der Temperatur des Kristallisators liegt. Durch die Einleitung des Kristallbreies in den Mischbehälter mit höherer Temperatur wird ein besonderer thermischer Streß erzeugt, der insbesondere die Auflösung kleinerer Kristalle begünstigt und so die geringe Rekristallisationsgeschwindigkeit der isomerenreinen Isocyanat-Kristalle kompensiert.

Der Mischbehälter, in den vorzugsweise auch die Ausgangs-Isomerenmischung eingeleitet wird, ersetzt also den bei üblichen Suspensionskristallisationsverfahren eingesetzten Rekristallisationsbehälter.

Je nach Größe des Mischbehälters und damit der Verweilzeit im Mischbehälter kann es zusätzlich zu einer Kristallvergrößerung durch Rekristallisation kommen, jedoch ist es erfindungsgemäß bevorzugt, einen Mischbehälter mit kleinem Volumen und relativ kurzer Verweilzeit vorzusehen. Insbesondere bevorzugt wird der Mischbehälter so dimensioniert, daß die Verweilzeit des Kristallbreis im Mischbehälter zwischen 1 und 15 Minuten beträgt, besonders bevorzugt zwischen 5 und 10 Minuten.

Erfindungsgemäß wesentlich ist, daß dem Mischbehälter Wärme zur Aufrechterhaltung der erhöhten Temperatur und zum Rufschmelzen der Kristalle zugeführt wird. Die Wärme kann über eine Mantelheizung des Mischbehälters indirekt zugeführt werden, oder aber bevorzugt durch entsprechende Temperierung der in den Mischbehälter ebenfalls einzuleitenden Ausgangs-Isomerenmischung.

Die Ausschleusung der an isomerenreinem Isocyanat verarmten Mutterlauge erfolgt durch ein zwischen Kristallisator und Mischbehälter vorgesehenes Filter.

Vorzugsweise wird als Ausgangs-Isomerenmischung eine Mischung aus 80 Gew.-% 2,4-TDI und 20 Gew.-% 2,6-TDI eingesetzt. Die Ausgangs-Isomerenmischung wird dem Mischbehälter vorzugsweise mit einer Temperatur zwischen 17 und 30°C, insbesondere bevorzugt zwischen 18 und 24°C zugeführt.

Die an 2,4-TDI abgereicherte Flüssigphase enthält noch 65 bis 69, vorzugsweise 66 bis 68 Gew.-% 2,4-TDI. Im Kristallisator wird eine Temperatur zwischen 5 und 7°C aufrechterhalten.

Im Kopf der Waschsäule wird 2,4-TDI mit einer Konzentration von oberhalb 99 %, bevorzugt oberhalb 99,8 %, insbesondere bevorzugt oberhalb 99,9 % bei einer Temperatur von mindestens 22°C entnommen.

Die in der Waschsäule abgetrennte Mutterlauge wird in den Kristallisator zurückgeführt.

Im Falle der Isomerentrennung von MDI wird vorzugsweise eine Ausgangsisomerenmischung von 70 bis 90 % 4,4'-MDI und 30 bis 10 % 2,4'-MDI eingesetzt. Die Ausgangsisomerenmischung wird dem Mischbehälter vorzugsweise mit einer Temperatur von 30 bis 45°C zugeführt. Die an 4,4'-MDI abgereicherte Flüssigkeit enthält vorzugsweise noch 50 bis 60 % 4,4'-MDI. Die Temperatur im Kristallisator beträgt vorzugsweise 15 bis 21°C.

Nach einer anderen Formulierung der Erfindung betrifft diese ein Verfahren zur Isomerentrennung von Isocyanaten, insbesondere TDI und MDI durch Suspensionskristallisation und Abtrennung isomerenreiner Kristalle, das dadurch gekennzeichnet ist, daß die Kristallsuspension nach dem Austritt aus dem Kristallisator mit der Ausgangsisomerenmischung vermischt wird, wobei die Ausgangsisomerenmischung mit einer solchen Temperatur zugeführt wird, daß die entstehende Mutterlauge eine relative Untersättigung von 4 bis 20 %, vorzugsweise 7 bis 17 % aufweist.

Dabei wird unter entstehender Mutterlauge die Mischung aus vom Kristallisator zugeführter Mutterlauge, die die Temperatur des Kristallisators und eine Restkonzentration an dem als Kristalle gewonnenen Isomeren aufweist, und der zugeführten Ausgangsisomerenmischung mit höherer Konzentration verstanden. Diese Mischung soll eine Konzentration aufweisen, die um 4 bis 20 % niedriger liegt, als die der Mischungstemperatur entsprechende Gleichgewichtskonzentration gemäß Liquidus-Kurve des Phasendiagramms. In dieser im Mischbehälter durch Vermischen erzeugten Mutterlauge befinden sich die Kristalle zunächst noch bei der niedrigeren Temperatur des Kristallisators. Kleinere Kristalle werden in der untersättigten Mutterlauge aufgelöst. Größere Kristalle werden zunächst angelöst, wodurch die Mutterlaugenkonzentration in der Grenzschicht ansteigt. Gleichzeitig entziehen die größeren Kristalle der sie umgebenden Mutterlauge so viel Wärme, daß diese in den Übersättigungsbereich gelangt. Die größeren Kristalle wachsen wieder.

Je größer die Untersättigung ist, um so mehr kleine Kristalle werden aufgelöst; je geringer die Untersättigung ist, desto stärker wachsen größere Kristalle. Die optimale Untersättigung hängt von der Kristallgrößenverteilung, die im Kristallisator erzeugt wird, ab. Sie kann durch Variation der Temperatur der zugeführten Ausgangsisomerenmischung in Abhängigkeit von der Trennleistung des Wäschers durch einfache Vorversuche ermittelt werden.

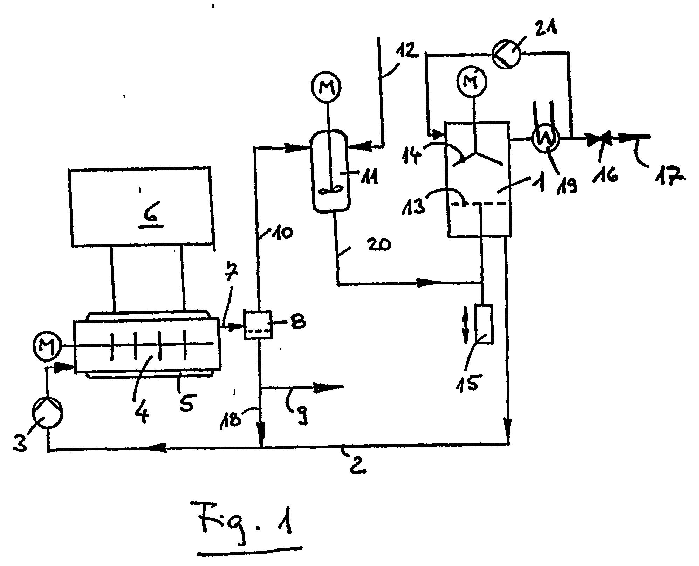

Die Erfindung wird nachfolgend anhand der beigefügten Figur 1 näher erläutert:

Aus der Waschkolonne 1 über Leitung 2 mittels Pumpe 3 rezirkulierte Mutterlauge wird in den Kristallisator 4, dessen Kühlmantel 5 aus dem Kühlaggregat 6 gespeist wird,- eingeleitet. Der Kristallbrei gelangt aus dem Kristallisator 4 über Leitung 7 in das Filter 8, wo abgereicherte Mutterlauge über Leitung 9 ausgeschleust wird. Der an Kristallen entsprechend angereicherte Kristallbrei wird über Leitung 10 in den Mischbehälter 11 eingeleitet, in dem eine höhere Temperatur herrscht als in dem Kristallisator 4. Ferner wird in den Mischbehälter 11 die Ausgangs-Isomerenmischung über Leitung 12 eingeleitet. Diese befindet sich auf einer ausreichenden Temperatur, um die Temperatur im Mischbehälter aufrecht zu erhalten. Alternativ kann der Mischbehälter 11 mit einer Mantelheizung versehen sein. Die aus dem Mischbehälter 11 austretende Mutterlauge, aus der kleinere Kristalle aufgeschmolzen wurden, gelangt über Leitung 20 in die Waschkolonne oberhalb der Siebplatte 13. Die Mutterlauge tritt durch die Siebplatte 13 hindurch und wird in den Kristallisator über Leitung 2 zurückgeführt. Die Siebplatte 13 wird mittels der Hubkolbenpumpe 15 periodisch gegen den Kristallschaber 14 bewegt, wobei die Mutterlauge aus dem Raum oberhalb der Siebplatte 13 heraus gedrückt wird und die Kristalle kompaktiert werden. Der Schaber 14 trägt von dem kompaktierten Kristallblock so viele Kristalle ab wie durch die Siebplatte 13 nachgeschoben werden. Die Kristalle werden im Wärmeaustauscher 19 aufgeschmolzen und über das Ventil 16 und Leitung 17 ausgetragen. Die aus dem Wärmeaustauscher 19 austretende Schmelze wird teilweise über Pumpe 21 in den Kopf der Waschsäule zurückgeführt, so daß dort eine Temperatur in der Nähe des Schmelzpunktes aufrechterhalten wird. Das Ventil 16 hält oberhalb der Siebplatte 13 zumindest einen solchen Druck aufrecht, daß ein Teil der aufgeschmolzenen Kristalle, d.h. der reinen Flüssigkeit, zur Abspülung noch anhaftender Mutterlauge durch den kompaktierten Kristallblock gedrückt wird. Ein Teil der im Filter 8 abgetrennten Mutterlauge kann über Leitung 18 in den Kristallisator 4 zurückgeführt werden, um den Feststoffgehalt im Kristallisator 4 zu steuern.

### Beispiel

In einer Pilotanlage gemäß Figur 1 werden über Leitung 12 stündlich 100 Teile TDI 80/20 mit einer Temperatur von 25°C eingeleitet. Über Leitung 9 werden stündlich 60,6 Teile TDI 67/33 und über Leitung 17 39,4 Teile 99,9 %iges 2,4-TDI ausgeschleust. Im Kristallisator 4 wird eine Temperatur von 6,3°C aufrecht erhalten. Der Kristallgehalt im Kristallisator 4 wird durch teilweise Rückführung der im Filter 8 abgetrennten Mutterlauge über Leitung 18 bei 25 Gew.-% gehalten. Nach Abtrennung eines Teils der Mutterlauge in Filter 8 gelangt der Kristallbrei, der eine Temperatur von ebenfalls 6,3°C aufweist, in den Mischbehälter 11. Der den Mischbehälter 11 verlassende und der Waschkolonne zugeführte Kristallbrei weist eine Temperatur von 11,5°C und eine Flüssigphasenkonzentration von 76,5 Gew.% 2,4-TDI auf. Die Verweilzeit im Mischbehälter 11 beträgt 10 Minuten, die Verweilzeit im Kristalüsator 4 120 Minuten.

## Patentansprüche

1. Verfahren zur Gewinnung von reinen Isocyanatisomeren aus einer Isocyanatisomeren-Mischung durch Suspensionskristallisation und Abtrennung isomerenreiner Kristalle, **dadurch gekennzeichnet, daß** vor der Abtrennung der Kristalle 5 bis 40 Gew.-% der erzeugten Kristalle unter gleichzeitiger Erhöhung der Temperatur des Kristallbreis um 4,5 bis 9°C wieder aufgeschmolzen werden und die Abtrennung der isomerenreinen Kristalle aus der Suspension unter Aufschmelzen in einer Waschsäule erfolgt.

2. Verfahren nach Anspruch 1, **dadurch gekennzeichnet, daß** 10 bis 24 Gew.-% der erzeugte Kristalle aufgeschmolzen werden.

3. Verfahren nach Anspruch 1 oder 2, **dadurch gekennzeichnet, daß** das Aufschmelzen und die Temperaturerhöhung des Kristallbreis in einem zwischen Kristallisationsbehälter und Abtrennvorrichtung vorgesehenen Mischbehälter erfolgt, dem Wärme zugeführt wird.

4. Verfahren nach einem der Ansprüche 1 bis 3, **dadurch gekennzeichnet, daß** die Ausgangsmischung dem Mischbehälter zugeführt wird.

5. Verfahren nach einem des Ansprüche 1 bis 4, **dadurch gekennzeichnet, daß** die Kristallsuspension nach dem Austritt aus dem Kristallisator mit der Ausgangsisomerenmischung vermischt wird, wobei die Ausgangsisomerenmischung mit einer solchen Temperatur zugeführt wird, daß die Mischung aus Ausgangsisomerenmischung und aus dem Kristallisator zugeführter Flüssigphase ohne Berücksichtigung der Kristalle untersättigt ist.

6. Verfahren nach einem der Ansprüche 1 bis 5, **dadurch gekennzeichnet, daß** zwischen Kristallisator und Mischbehälter ein Filter vorgesehen ist, mit dem die an dem zu gewinnenden reinen Isomeren abgereicherte Flüssigphase ausgeschleust wird.

7. Verfahren nach einem der Ansprüche 1 bis 6, **dadurch gekennzeichnet, daß** die im Mischbehälter erforderliche Wärme durch Temperierung der zugeführten Ausgangsmischung zugeführt wird.

8. Verfahren nach einem der Ansprüche 1 bis 7, **dadurch gekennzeichnet, daß** als Ausgangsmischung eine Mischung aus 80 Gew.-% 2,4-TDI und 20 Gew.-% 2,6-TDI eingesetzt wird.

9. Verfahren nach einem der Ansprüche 1 bis 8, **dadurch gekennzeichnet, daß** die an 2,4-TDI abgereicherte ausgeschleuste Flüssigphase 65 bis 69 Gew.-% 2,4-TDI enthält.

10. Verfahren nach einem der Ansprüche 1 bis 9, **dadurch gekennzeichnet, daß** die Verweilzeit im Mischbehälter 1 bis 15 Minuten beträgt.

## Claims

1. Process for obtaining pure isocyanate isomers from a mixture of isocyanate isomers by suspension crystallisation and separation of isomerically pure crystals, **characterised in that** prior to the separation of the crystals, 5 to 40 wt.% of the crystals produced are melted again while the temperature of the crystalline substance is simultaneously increased by between 4.5°C and 9°C, and the separation of the isomerically pure crystals from the suspension with melting is carried out in a washing column.

2. Process according to claim 1, **characterised in that** 10 to 24 wt.% of the crystals produced are melted.

3. Process according to claim 1 or 2, **characterised in that** the melting of the crystalline substance and the raising of its temperature are carried out in a mixing tank placed between the crystallising tank and the separating device and supplied with heat.

4. Process according to one of claims 1 to 3, **characterised in that** the initial mixture is passed to the mixing tank.

5. Process according to one of claims 1 to 4, **characterised in that** the suspension of crystals, after having left the crystallising tank, is mixed with the initial mixture of isomers, the initial mixture of isomers being introduced at a temperature such that the mixture comprising the initial mixture of isomers and the liquid phase introduced from the crystallising tank is undersaturated, not taking account of the crystals.

6. Process according to one of claims 1 to 5, **characterised in that** a filter, through which the liquid phase depleted of the pure isomers being obtained is withdrawn, is placed between the crystallising tank and the mixing tank.

7. Process according to one of claims 1 to 6, **characterised in that** the heat required in the mixing tank is supplied by tempering the initial mixture introduced.

8. Process according to one of claims 1 to 7, **characterised in that** the initial mixture used is a mixture of 80 wt.% 2,4-TDI and 20 wt.% 2,6-TDI.

9. Process according to one of claims 1 to 8, **characterised in that** the withdrawn liquid phase depleted of 2,4-TDI contains 65 to 69 wt.% 2,4-TDI.

10. Process according to one of claims 1 to 9, **characterised in that** the residence time in the mixing tank is 1 to 15 minutes.

## Revendications

1. , Procédé pour isoler des isocyanates isomères purs à partir d'un mélange d'isocyanates isomères par cristallisation en suspension et séparation de cristaux d'isomère pur, **caractérisé en ce que**, avant la séparation des cristaux, on refond de 5 à 40 % en poids des cristaux formés par augmentation simultanée de 4,5 à 9°C de la température de la bouillie cristalline et on sépare les cristaux d'isomère pur de la suspension, avec fusion, dans une colonne de lavage.

2. Procédé selon la revendication 1, **caractérisé en ce que** l'on refond de 10 à 24 % en poids des cristaux formés.

3. Procédé selon la revendication 1 ou 2, **caractérisé en ce que** la fusion et l'augmentation de température de la bouillie cristalline sont réalisées dans un récipient de mélange prévu entre le récipient de cristallisation et l'appareil de séparation et auquel on apporte de la chaleur.

4. Procédé selon l'une des revendications 1 à 3, **caractérisé en ce que** le mélange initial est envoyé au récipient de mélange.

5. Procédé selon l'une des revendications 1 à 4, **caractérisé en ce que** la suspension de cristaux, après sortie du cristalliseur, est mélangée avec le mélange initial d'isomères, ce dernier étant amené à une température telle que le mélange du mélange initial d'isomères et de la phase liquide amenée au cristalliseur, non tenu compte des cristaux, soit au-dessous de la saturation.

6. Procédé selon l'une des revendications 1 à 5, **caractérisé en ce que**, entre le cristalliseur et le récipient de mélange, on a prévu un filtre qui permet d'évacuer le liquide appauvri en l'isomère pur recherché.

7. Procédé selon l'une des revendications 1 à 6, **caractérisé en ce que** la chaleur nécessaire pour le récipient de mélange est apportée par chauffage du mélange initial amené.

8. Procédé selon l'une des revendications 1 à 7, **caractérisé en ce que** le mélange initial mis en oeuvre est à 80 % en poids de 2,4-TDI et 20 % en poids de 2,6-TDI.

9. Procédé selon l'une des revendications 1 à 8, **caractérisé en ce que** la phase liquide évacuée, appauvrie en 2,4-TDI, contient 65 à 69 % en poids de ce dernier.

10. Procédé selon l'une des revendications 1 à 9, **caractérisé en ce que** la durée de passage dans le récipient de mélange est de 1 à 15 min.
